Europäisches Patentamt

European Patent Office

Office européen des brevets

(19)

(11) Publication number: **0 063 417**
**B1**

(12) **EUROPEAN PATENT SPECIFICATION**

(45) Date of publication of patent specification: **13.11.85**

(51) Int. Cl.⁴: **A 61 M 1/00,** A 61 M 7/00

(21) Application number: **82301540.9**

(22) Date of filing: **24.03.82**

(54) Surgical evacuator.

(30) Priority: **02.04.81 AU 8270/81**

(43) Date of publication of application:
**27.10.82 Bulletin 82/43**

(45) Publication of the grant of the patent:
**13.11.85 Bulletin 85/46**

(84) Designated Contracting States:
**AT BE CH DE FR GB IT LI LU NL SE**

(56) References cited:
**CH-A- 117 446**
**FR-A-1 270 595**
**GB-A- 125 050**

(73) Proprietor: **Wilson, John David**
**29 Woolwich Road**
**Hunters Hill New South Wales 2110 (AU)**

(72) Inventor: **Wilson, John David**
**29 Woolwich Road**
**Hunters Hill New South Wales 2110 (AU)**

(74) Representative: **Topps, Ronald et al**
**D. YOUNG & CO 10 Staple Inn**
**London WC1V 7RD (GB)**

Courier Press, Leamington Spa, England.

## Description

In the surgical treatment of patients (whether human or animal) it is frequently necessary to evacuate detrimental fluid accumulates from a cavity or sac in the patient's body. The cavity or sac may be a wound (caused by surgery or otherwise) which has been closed at its outermost region by suturation yet is required to heal from its innermost region in the direction towards the outermost region, or it may be a natural sac such as the bladder, a lung, the stomach or the like.

In many cases nothing more than a simple drainage tube, duct or catheter is required, but in others the fluid to be drained may contain blood clots, mucus, or other matter less fluent than the remainder of the accumulate. When this is so, the intake end of an ordinary drainage tube will frequently become occluded by the less fluent matter and it then becomes necessary to apply a substantially greater suction to the drainage tube in order to induce the obstructing matter to enter the tube and proceed to waste by way thereof; moreover, to facilitate evacuation, it is often desirable to irrigate the cavity or sac to be drained, with a saline or other solution to act as a vehicle for the matters to be evacuated.

Although the invention is applicable to a variety of drainage uses as indicated above the need for an effective in-line evacuator arose in connection with the removal of blood clots from the bladder following prostatic or other surgery related to urological disorders. Because of this, the invention is described herein largely in terms of a urological in-line evacuator.

In the prior art, it has been common to use what is known as the cystoflo drainage system. This prior system comprises a catheter whereof the proximal end portion is entered into the bladder and the distal end portion opens to a urinary drainage bag or other receiver for the drained matter. The catheter of such a system includes a compressible rubber bulb which can be stocked with an irrigating liquid and delivered into the bladder by compression of the bulb. For suctional purposes, pressure on the bulb is relaxed so that of its own elasticity, the bulb tends to resume its unsqueezed globular shape. Experience has shown that the prior system just discussed fails to develop a sufficiently strong suctional effect and frequently becomes quite ineffective in the presence of heavy bleeding involving extensive clotting. When that happens, it is necessary to break the closed in-line system, thereby risking loss of its necesary sterility, and endeavour to evacuate the clots by use of other means. Even if this endeavour is successful it still involves another risk of sterility loss in re-establishing the broken in-line system.

GB—A—125050 discloses a pump which can be used to evacuate a collection of pathological liquid by suction. It essentially consists of a piston and cylinder device with one end of the cylinder being connected to a boss provided with a cock which is manually moved by an operating ring and rod system and the piston rod is hollow and has its outer end provided with a cock which is manually moved by an operating ring. Evacuation is achieved by opening the cock provided on the boss, closing the cock provided on the piston and moving the piston rod and thus the piston to draw the liquid into the cylinder. The cock provided on the boss is then closed and the cock on the piston rod is opened and the piston rod and piston moved in the opposite direction to expell the liquid from the cylinder through the piston rod. The provision of manually operable cocks has the disadvantage that they can inadvertently be left in a closed condition when the use of the pump requires them to be open.

The object of the present invention is to overcome the indicated shortcomings in the provision of an in-line evacuator which:

(1) provides a more-effectively powerful evacuation suction;

(2) will avoid the necessity, to break the closed and sterile in-line system and thus reduce the likelihood of introducing infection; and

(3) will remove need for catheter irrigation trays which are costly to set up and not always readily available when needed urgently.

According to the present invention there is provided a surgical in line evacuator comprising a cylindrical chamber, a piston plunger reciprocable longitudinally of said chamber, an open ended tubular piston rod upon one end of which said piston plunger is mounted, an intake tube extending from the end of the chamber opposite to the piston rod and which can be introduced into a body cavity or sac to be drained, and a drain tube leading to waste and extending from the other end of said piston rod, an irrigation liquid inlet opening adjacent said end of the chamber opposite to the piston rod and a supply tube extending from said inlet; said tubes being resiliently deformable so as to permit distortion by manually applied pressure to effect flow control therethrough and automatic reversion to their original undeformed shapes to present unobstructed bores on the removal of such pressure, characterised in that there is no other means for obstructing flow through the intake tube, piston rod and drain tube and that the irrigation liquid inlet opens into said chamber.

An embodiment of the invention will now be described, by way of an example, with reference to the accompanying drawings, in which:—

Figure 1 is a purely diagrammatic representation of a urological in-line evacuator according to the present invention, and

Figures 2 to 7 are schematic representations of the cylinder and plunger arrangement shown in Figure 1, showing the parts thereof disposed in accordance with several stages in usage.

Referring to the drawings, the evacuator comprises an intake tube 8 which can be introduced into the bladder indicated at 9. The tube 8 may conveniently be in one or more separable parts as will be understood. The evacuator further comprises a drain tube 10 from which evacuated liquid may be discharged in any convenient manner, for example, by being sent into a common urinary drainage receptacle 11. A cylindrical chamber 12 is connected in line with the tubes 8 and 10, the chamber 12 having one end 13 open to the tube 8 and housing a piston plunger 14 which is reciprocable longitudinally of chamber 12. Plunger 14 is mounted on a tubular piston rod 15, the arrangement being such that the piston rod 15 has one end 16 open to that end of the chamber 12 which is in communication with the intake tube 8. The piston rod 15 slides in a bearing 17 and has its other or distal end 18 open to the drain tube 10. An irrigation inlet 19 opens to the chamber 12 and is connected by a supply tube 20 with a source of irrigation liquid indicated at 21.

That portion of the tube 8 which is inserted into the bladder 9 by way of the urethra 22 may be of the common type known as a "Foley" balloon catheter as indicated as 23.

When the evacuator is required to be used in the manner of a simple drainage tube, the piston plunger 14 is located as shown in Figure 2 so that it closes the irrigation inlet 19. This inlet 19 could be closed by furnishing it with an on-off cock, but closure by way of the plunger 14 is preferred since the apparatus is thus less complicated.

The evacuator (as shown in Figure 2), operates in simple drainage fashion like an ordinary catheter.

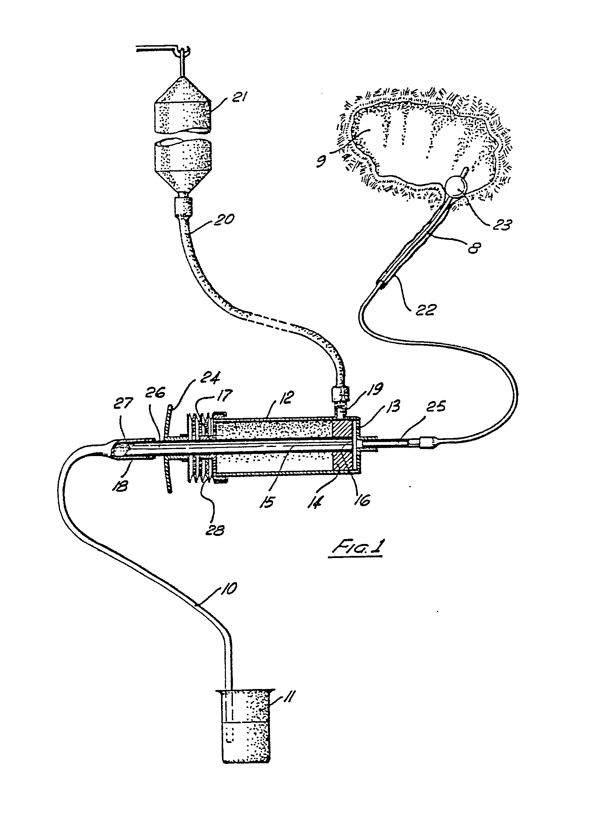

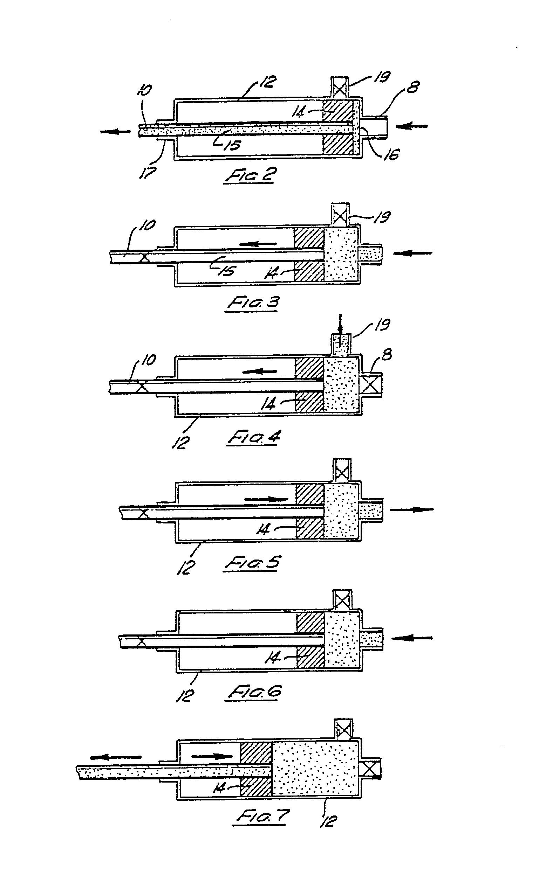

In some cases it may be required to increase the suction in the tube 8 without irrigation being immediately necessary. If so, inlet 19 and drain tube 10 are closed, and plunger 14 retracted as indicated in Figure 3. This retraction is effected by finger pull on a handpiece 24 (Figure 1) fixed on the piston rod 15.

Preferably tubes 8, 10 and 20 are each pliantly resilient and their closure may be effected by manually squeezing or bending them. Such an arrangement is greatly preferred by comparison for example with control valves since there is a risk that valves may be inadvertently left in "off" or closed position when not required; moreover, such valves add unnecessary complication in the apparatus as a whole. For preference, each of the tubes 8, 10 and 20 is made of rubber, plastics or like flexible material so that the passage, or passages, concerned may be closed simply by hand or finger pressure sufficient to bend the tube or tubes to be closed so as to halt fluid flow through it or them. This has the advantage that when any one of the tubes 8, 10 or 20 is required to revert to open position, it will do so of its own accord (due to the resilience of the material from which it is made) immediately following relaxation of the hand or finger pressure which caused it to fold into closed position.

In the case of passage 19, it is effectively closed by the plunger 14 when the plunger 14 is in register with inlet 19 as shown in Figure 2. When the plunger 14 is not in that position, inlet 19 may be closed (as shown in Figures 3, 5, 6 and 7) by folding tube 20 effected by way of the user's thumb pressure.

The closure of drain tube 10 by the application of manual pressure may be facilitated by providing end 26 of piston rod 15 with a flat, oblique end 27 against which the adjacent portion of tube 10 can be flatly pressed by the palm of the hand.

When the bladder 9 is to be irrigated, cylinder 12 (see Figure 4) is charged with irrigation liquid by closing tube 8 and 10, opening passage 19 and retracting the plunger 14. To deliver the charge of liquid, tube 10 remains closed, passage 19 is closed, tube 8 opened and the plunger 14 advanced as shown in Figure 5.

If irrigation is followed by high suction evacuation, as indicated in Figure 6, the apparatus is conditioned and operated in the same way as explained in connection with Figure 3. Following such evacuation the contents of the cylinder 14 are expelled, as shown in Figure 7, and the apparatus reverted to ordinary catheter drainage as shown in Figure 2.

As a further safeguard against loss of internal sterility, the space between bearing 17 and handpiece 24 may be sealed by use of a conventional syphon or concertina sleeve as indicated at 28 in Figure 1.

It will be appreciated that further modifications may be applied to the evacuator as described above. For example, the irrigation inlet 19 may be placed in the end wall 13 of the chamber 12 or otherwise as may be convenient provided it is closable (by plunger 14 or otherwise) when required. Again, a compression loading spring may be applied between handpiece 24 and the adjacent end of the chamber 12 so that the evacuator may be used as a self-acting wound drain. Such a spring could be incorporated in, or constituted by, a concertina sleeve such as that indicated at 28; or, in the event of such a sleeve 28 not being incorporated, by an ordinary helical spring sleeved on piston rod 15 between the handpiece 24 and the adjacent end cover for chamber 12. If a concertina sleeve such as 28 is required to be employed with a spring as just discussed, the spring may be closely sleeved about rod 15 between bearing 17 and the mounting boss 29 of handpiece 24; that is, inside a sleeve such as 28.

It will be further appreciated that in some cases it may be desirable, for safety or other reasons, to lock the plunger 14 in its closed position (as shown in Figure 1) so to ensure against unwanted discharge of liquid from container 21. This may be accomplished by use of a common grub screw able to bear upon rod 15 through bearing 17, or by a link pivoted on cylinder 12 and able to engage handpiece 24, or otherwise.

**Claims**

1. A surgical in-line evacuator comprising a cylindrical chamber (12), a piston plunger (14) reciprocable longitudinally of said chamber (12), an open ended tubular piston rod (15) upon one end of which said piston plunger (14) is mounted, an intake tube (8) extending from the end of the chamber (12) opposite to the piston rod and which can be introduced into a body cavity or sac (9) to be drained, a drain tube (10) leading to waste and extending from the other end of said piston rod (15), an irrigation liquid inlet (19) opening (12) adjacent said end of the chamber opposite to the piston rod and a supply tube (20) extending from said inlet; said tubes (8, 10, 20) being resiliently deformable so as to permit distortion by manually applied pressure to effect flow control therethrough and automatic reversion to their original undeformed shapes to present unobstructed bores on the removal of such pressure, characterised in that there is no other means for obstructing flow through the intake tube (8), piston rod (15) and drain tube (10) and that the irrigation liquid inlet (19) opens into said chamber (12).

2. An evacuator according to claim 1, further characterised in that said other end of the tubular piston rod (15) terminates in an obliquely disposed flat face (27) against which a part of the resilient drain tube (10) is able to seat flatly.

3. An evacuator as claimed in claim 1 or claim 2, further characterised in that the end portion of said piston rod (15) external of the chamber (12) is sealed thereto by way of a syphon sleeve (28), ensuring that an initially sterile system remains so.

4. An evacuator as claimed in any one of the preceding claims, further characterised in that the piston rod (15) is provided with a hand-piece (24) by which the piston rod (15) can be moved.

## Revendications

1. Evacuateur chirurgical en ligne comprenant une chambre cylindrique (12), un piston (14) pouvant effectuer un déplacement longitudinal de va-et-vient dans ladite chambre (12), une tige de piston tubulaire ouverte à une extrémité (15), sur une extrémité de laquelle est monté ledit piston (14), un tube d'admission (8) qui part de l'extrémité de la chambre (12) opposée à la tige de piston et qui peut être introduit dans une cavité corporelle ou une poche (9) à drainer, un tube de drainage (10) conduisant à l'évacuation des déchets, qui part de l'autre extrémité de ladite tige de piston (15), un orifice d'admission de liquide d'irrigation (19) débouchant au niveau de ladite extrémité de la chambre opposée à la tige de piston et un tube d'alimentation (20) partant dudit orifice d'admission; lesdits tubes (8, 10, 20) étant élastiquement déformables pour permettre leur déformation par pression manuelle pour exercer une commande de l'écoulement qui les traverse et un retour automatique à leurs formes naturelles non déformées pour présenter des passages non obstrués au relâchement de cette pression, caractérisé en ce qu'il n'y a pas d'autre moyen pour entraver l'écoulement qui traverse le tube d'admission (8), la tige de piston (15) et le tube de drainage (10), et en ce que l'orifice d'admission du liquide d'irrigation (19) débouche dans ladite chamber (12).

2. Evacuateur selon la revendication 1, caractérisé en outre en ce que l'autre extrémité de la tige de piston tubulaire (15) se termine par une face plate disposée obliquement (27) contre laquelle une portion du tube de drainage élastique (10) peut s'appliquer.

3. Evacuateur selon la revendication 1 ou la revendication 2, caractérisé en outre en ce que la partie terminale de ladite tige de piston (15) à l'extérieur de la chambre (12) est fixée hermétiquement à la chambre au moyen d'un manchon à soufflet (28) garantissant qu'un dispositif initialement stérile le demeurera.

4. Evacuateur selon l'une quelconque des revendications ci-dessus, caractérisé en outre en ce que la tige de piston (15) est équipée d'une poignée (24) au moyen de laquelle la tige de piston (15) peut être déplacée.

## Patentansprüche

1. Chirurgisches in-line Entleerungsgerät mit einer zylindrischen Kammer (12), einem in Längsrichtung der genannten Kammer (12) hin- und herbewegbaren Plungerkolben (14), einer beidseitig offenen rohrförmigen Kolbenstange (15), an deren einem Ende der genannte Plungerkolben (14) angebracht ist, mit einem Einlaßrohr (8), das sich von dem der Kolbenstange gegenüberliegenden Ende der Kammer (12) erstreckt und in einen zu entleerenden Körperhohlraum oder Sack eingeführt werden kann, einem Ableitungsrohr (10), das zu einem Abfluß führt und sich vom anderen ende der genannten Kolbenstange (15) erstreckt, mit einem Einlaß (19) für Irrigationsflüssigkeit, der nahe dem der Kolbenstange gegenüberliegenden genannten Ende der Kammer mündet, und einem Versorgungsrohr (20), das sich vom genannten Einlaß weg erstreckt, wobei die genannten Rohre (8, 10, 20) elastisch verformbar sind, um bei händischem Druck eine Formveränderung für die Regelung der Durchflußwege und eine automatische Rückkehr in den ursprünglichen nicht verformten Zustand zu ermöglichen, sodaß bei Entfernen eines solchen Druckes die Durchgänge frei werden, dadurch gekennzeichnet, daß keine anderen Mittel zum Unterbinden des Durchflusses durch das Einlaßrohr (8), die Kolbenstange (15) und das Ableitungsrohr (10) vorgesehen sind, und daß der Einlaß (19) für die Irrigationsflüssigkiet in die genannte Kammer (12) mündet.

2. Entleerungsgerät nach Anspruch 1, weiters dadurch gekennzeichnet, daß das genannte andere Ende der rohrförmigen Kolbenstange (15) in einer schräg angeordneten, glatten Fläche (27) mündet, an der ein Teil des elastischen Ableitungsrohres (10) flach aufsitzen kann.

3. Entleerungsgerät nach Anspruch 1 oder 2, weiters dadurch gekennzeichnet, daß der Endabschnitt der genannten Kolbenstange (15) außerhalb der Kammer (12) mit dieser durch eine Faltenhülse (28) dichtend verbunden ist, um zu gewährleisten, daß die Sterilität des Systems erhalten bleibt.

4. Entleerungsgerät nach einem der vorhergehenden Ansprüche, weiters dadurch gekennzeichnet, daß die Kolbenstange (15) mit einem Handstück (24) versehen ist, mit dem die Kolbenstange (15) bewegt werden kann.

*Fig.1*

Fig. 2

Fig. 3

Fig. 4

Fig. 5

Fig. 6

Fig. 7